# EUROPEAN PATENT SPECIFICATION

(11) **EP 2 691 034 B1**
(45) Date of publication and mention of the grant of the patent: **10.08.2016**
(21) Application number: 12763063.0
(22) Date of filing: 30.03.2012
(51) Int. Cl.: A61B 17/285, A61B 17/32, A61B 17/00

(54) **ULTRASONIC SURGICAL INSTRUMENT**
CHIRURGISCHES ULTRASCHALLINSTRUMENT
INSTRUMENT CHIRURGICAL ULTRASONORE

(30) Priority: 30.03.2011 US 201161469582 P
(43) Date of publication of application: 05.02.2014
(73) Proprietor: Covidien LP, Mansfield, MA 02048 (US)
(72) Inventor: CUNNINGHAM, James, S., Boulder, CO 80304 (US); DICKHANS, William, J., Longmont, CO 80503 (US); HEMPSTEAD, Russell, D., Lafayette, CO 80026 (US); KAPPUS, John, J., Denver, CO 80218 (US); KERR, Duane, E., Loveland, CO 80537 (US); LARSON, Eric, R., Boulder, CO 80301 (US); NAU, William, H., Longmont, CO 80504 (US); ROSS, Anthony, B., Boulder, CO 80301 (US); STODDARD, Robert, B., Steamboat Springs, CO 80487 (US)
(74) Representative: Soames, Candida Jane
(86) International application number: PCT/US2012/031601
(87) International publication number: WO 2012/135715

(56) References cited:
- EP-A1- 1 518 505
- WO-A1-2012/061645
- WO-A2-2011/008672
- US-A- 6 129 735
- US-A1- 2004 097 911
- US-A1- 2005 143 769
- US-A1- 2005 192 610
- US-A1- 2007 282 332
- US-A1- 2009 030 311
- US-A1- 2009 030 439
- US-A1- 2009 143 806
- US-A1- 2009 182 365
- US-A1- 2009 264 909
- US-A1- 2010 179 545
- US-B1- 6 193 709
- US-B1- 6 887 252
- US-B1- 6 887 252

## Description

### BACKGROUND

### Technical Field

The present disclosure relates to ultrasonic surgical instruments. More particularly, the present disclosure relates to ultrasonic surgical instruments including at least one jaw member configured to support tissue during ultrasonic treatment thereof.

### Description of Related Art

Ultrasonic energy-powered instruments configured to cut and/or fragment tissue are known in the art. Ultrasonic instruments, typically, include a transducer that is coupled to a probe/waveguide having an active member (e.g., cutting blade, shear, hook, ball, etc.) at a distal end thereof. In use, ultrasonic energy is utilized to vibrate (e.g., at frequency usually in the range of 20 KHz to 60 KHz) the active member to treat tissue of interest.

Ultrasonic instruments may include any of a variety of probe configurations to achieve a specific surgical result. For example, the probe configuration may include an active member in the form of a cutting blade that is combined with a movable jaw configured to grasp and/or manipulate tissue. In certain instances, a tissue contacting surface (which is typically made from metal) of the movable jaw member may include a polytetrafluoroethylene (PTFE) liner configured to prevent the cutting blade from coming into contact with the tissue contacting surface. Such ultrasonic instruments are primarily used in a variety of medical procedures including open surgical procedures, luminal procedures, and endoscopic procedures.

During use, the movable jaw member provides support for tissue as the cutting blade vibrates to treat tissue. The PTFE liner and/or the tissue contacting surface of the movable jaw member may wear as a result of prolonged use. As can be appreciated, wear of the PTFE liner and/or the tissue contacting surface of the movable jaw member may result in a decreased surgical effect to tissue. That is, as the PTFE liner and/or tissue contacting surface wears, its tissue supporting capabilities may be diminished. EP 1518505 is considered to be the closet prior art and discloses the preamble of independent claim 1.

### SUMMARY

In view of the foregoing, ultrasonic instruments including at least one jaw member configured to support tissue during ultrasonic treatment thereof may prove useful in the medical art.

Embodiments of the present disclosure are described in detail with reference to the drawing figures wherein like reference numerals identify similar or identical elements. As used herein, the term "distal" refers to a portion that is being described which is further from a user, while the term "proximal" refers to a portion that is being described which is closer to a user.

An aspect of the present disclosure provides an ultrasonic surgical instrument. The ultrasonic surgical instrument includes a housing having an elongated shaft extending therefrom. The shaft defines a longitudinal axis therethrough and has a jaw member disposed at a distal end thereof. The jaw member is movable between an open configuration and a clamping configuration. The jaw member including a tissue contacting surface having at least one laminate liner disposed thereon. A cutting blade extends from a distal end of the shaft and operably coupled to the housing and adjacent the jaw member to treat tissue. The at least one laminate liner is further defined by a plurality of laminate liners. Each laminate liner is stacked upon each other and selectively removable from the jaw member.
The plurality of laminate liners may include at least one substrate and at least one low durometer, low friction material. The at least one substrate may be formed from metal or ceramic. The at least one low durometer, low friction material may be formed frompolytetrafluoroethylene or silicone. Moreover, the at least one substrate may be coated with the at least one low durometer, low friction material.

At least one resilient member may be operably coupled to the jaw member and configured to bias the plurality of laminate liners in a generally outwardly configuration to facilitate removing each laminate liner of the plurality of liners. The at least one resilient member may be spring.

The plurality of laminate liners includes at least one low durometer, low friction material. The at least one low durometer, low friction material may be formed from polytetrafluoroethylene or silicone.

At least one resilient member may be operably coupled to the jaw member and configured to bias the plurality of laminate liners in a generally outwardly configuration to facilitate removing each laminate liner of the plurality of liners. The at least one resilient member may include a spring.

### BRIEF DESCRIPTION OF THE DRAWING

Various embodiments of the present disclosure are described hereinbelow with references to the drawings, wherein:
Fig. 1 is a right, perspective view of an ultrasonic instrument according to an embodiment of the present disclosure;
Fig. 2 is an enlarged, side, schematic view of a jaw member and a cutting blade, which does not comprise plural laminate liners as required by the claims;
Fig. 3 is an enlarged, side, schematic view of a jaw member and a cutting blade configured for use with the ultrasonic instrument depicted in Fig. 1, which does not comprise plural laminate liners as required by the claims;
Fig. 4 is an enlarged, side, schematic view of a jaw member and a cutting blade configured for use with the ultrasonic instrument depicted in Fig. 1, which does not comprise plural laminate liners as required by claim 1;
Fig. 5 is an enlarged, side, schematic view of a jaw member and a cutting blade configured for use with the ultrasonic instrument depicted in Fig. 1 according to an embodiment of the claims; and
Fig. 6 is an enlarged, side, schematic view of a jaw member and a cutting blade configured for use with the ultrasonic instrument depicted in Fig. 1 according to still yet another embodiment of the claims.

### DETAILED DESCRIPTION

Detailed embodiments of the present disclosure are disclosed herein; however, the disclosed embodiments are merely examples of the disclosure, which may be embodied in various forms. Therefore, specific structural and functional details disclosed herein are not to be interpreted as limiting, but merely as a basis for the claims and as a representative basis for teaching one skilled in the art to variously employ the present disclosure.

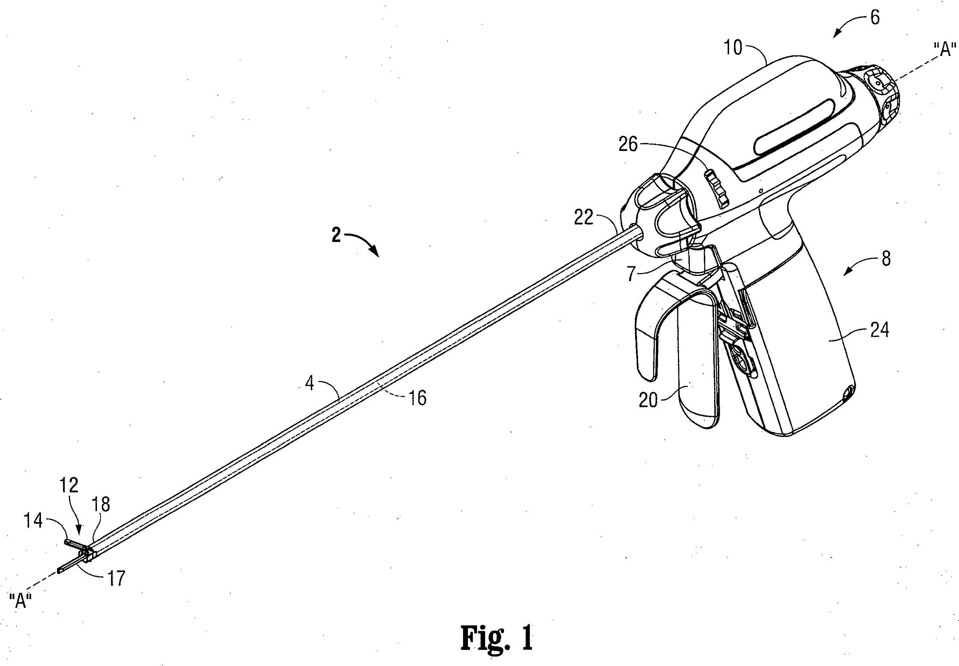

Turning now to Fig. 1, an ultrasonic surgical instrument 2 (instrument 2) according to an embodiment of the present disclosure is illustrated. In the illustrated embodiments, instrument 2 is described herein as being battery powered. Alternatively, instrument 2 may be externally powered, e.g., via a remote ultrasonic generator that couples to instrument 2.

Briefly, instrument 2 includes a housing 6 configured to house one or more components, e.g., transducer (not explicitly shown), a probe 16, and electrical circuitry that is configured for electrical communication with a battery assembly 8 of instrument 2. A proximal end of housing 6 is configured to releasably couple to an ultrasonic generator 10 and battery assembly 8. A distal end of housing 6 is configured to support and/or couple to a proximal end 22 of a shaft 4 having a longitudinal axis "A-A" defined therethrough. A rotation knob 26 operably couples to housing 6 and is configured to rotate shaft 4 approximately 360° in either direction about the longitudinal axis "A-A." Generator 10 includes the transducer that is coupled to probe 16 via a torque adapter (not explicitly shown) and configured to produce vibratory motion of a cutting blade 17 (Figs. 1-2) disposed at a distal end of probe 16 when a trigger 7 is depressed. This vibratory motion of cutting blade 17 is utilized to treat tissue of interest. Battery assembly 8 includes a handpiece 24 having a battery (not explicitly shown) operably disposed therein.

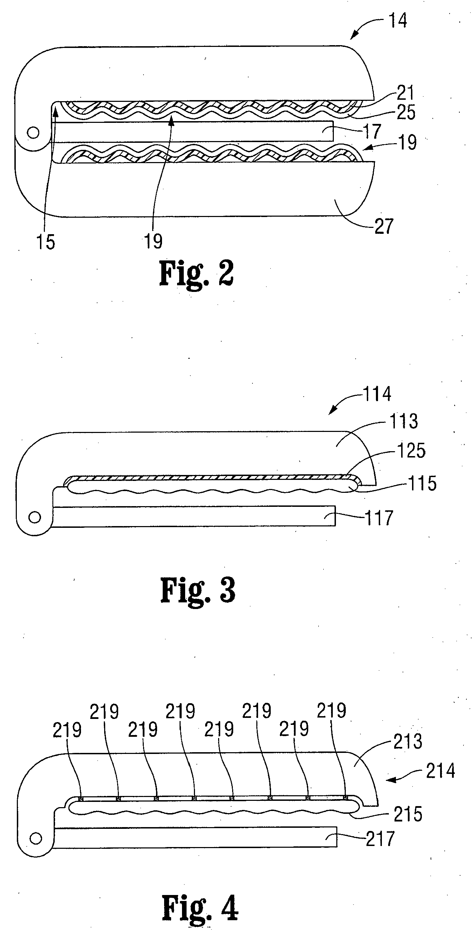

With reference to Figs. 1-2, an end effector 12 includes a first jaw member 14 (Fig. 1) that is supported at a distal end 18 of shaft 4 adjacent cutting blade 17. Jaw member 14 may be pivotably supported at the distal end of the shaft 4 via a pivot pin (not shown) and functions as a "clamping jaw." In particular, jaw member 14 is movable relative to cutting blade 17 (and/or the distal end 18 of the shaft 4) between an open configuration (Fig. 1) and a clamping configuration (Fig. 2) to clamp tissue when a lever or movable handle 20 (Fig. 1) is actuated. Jaw member 14 and cutting blade 17 are configured to collectively grasp and ultrasonically treat tissue. In particular, with tissue positioned between jaw member 14 and cutting blade 17, the cutting blade is configured to vibrate at a specific frequency (e.g., at a frequency in the range from about 20 KHz to about 60 KHz) to treat tissue.

Continuing with reference to Fig. 2, an example of jaw member 14 is illustrated including a jaw housing 13 having a tissue contacting surface 15 operably coupled thereto. Tissue contacting surface 15 provides a compliant, temperature resistant and low friction surface for cutting blade 17 when the jaw member 14 is in the clamping configuration and cutting blade 17 is treating tissue, i.e., vibrating. With this purpose in mind, tissue contacting surface 15 includes one or more laminate liners 19 disposed thereon.

Laminate liner(s) 19 may be formed from any suitable material including, but not limited to metal, ceramic, polymer, or combinations thereof. In the example illustrated in Figs. 1-2, laminate liner 19 includes a substrate 21 formed from one or more suitable materials, e.g., metal, ceramic, etc. Substrate 21 provides structural integrity for laminate liner 19 when jaw member 14 is in the clamping configuration and cutting blade 17 is treating tissue. Laminate liner 19 also includes one or more low durometer, low friction materials, e.g., polytetrafluoroethylene, silicone, and the like. In one particular embodiment, for example, substrate 21 may be coated or overmolded with a relatively thin coating of polytetrafluoroethylene or silicone 25.

In an example, it may prove useful to provide a second jaw member 27. In this particular example, second jaw member 27 may also include one or more laminate liners 19 (and operative components associated therewith) thereon configured to provide the same function as described above with respect to jaw member 14.

With reference again to Figs. 1-2, cutting blade 17 is configured to treat the tissue of interest and may be formed from any suitable material, including but not limited to, metal, ceramic, or other suitable material. In the illustrated example, cutting blade 17 may be formed from stainless steel or titanium. Metals of this type are suitable for forming cutting blade 17 because of their ability to withstand high temperatures and vibrations that are, typically, associated with cutting blade 17 during operation thereof.

During use of one particular example of the instrument 2, tissue may be positioned between the jaw member 14 and cutting blade 17. Subsequently, trigger 7 may be depressed to activate the cutting blade 17 to treat tissue of interest.

As cutting blade 17 vibrates against tissue contacting surface 15 of jaw member 14 and treats tissue, the relatively flexible (or compliant) nature of the laminate liner 19 reduces shear stresses against tissue contacting surface 15 caused by the vibrations of cutting blade 17.

The unique configuration of jaw member 14 including laminate liner 19 thereon allows the cutting blade 17 to treat tissue for prolonged periods of time without the likelihood of the tissue contacting surface 15 wearing or breaking down. As a result thereof, the operative life of the jaw member 15 (and/or cutting blade 17) is increased when compared to jaw members (and/or cutting blades) associated with conventional ultrasonic instruments.

From the foregoing and with reference to the various figure drawings, those skilled in the art will appreciate that certain modifications can also be made to the present disclosure without departing from the scope of the same. For example, while jaw member 14 has been described herein as including a laminate 19, other methods may be utilized to reduce shear stresses against tissue contacting surface 15.

With reference to Fig. 3, a jaw member 114 according to an alternate example is illustrated. Jaw member 114 is configured for use with instrument 2 and configured substantially similar to that of jaw member 14. In view thereof, only those features unique to jaw member 14 are described herein.

A laminate liner is operably coupled to jaw member 114 and includes a layer 125 having one or more low durometer, low friction materials, e.g., polytetrafluoroethylene, silicone, and the like. In particular, unlike laminate liner 19, laminate liner operably couples to jaw housing 113 and tissue contacting surface 115 and is positioned therebetween such that layer 125 is coupled to tissue contacting surface 115. Coupling laminate liner to jaw member 114 in this manner allows tissue contacting surface 115 to move in concert with cutting blade 117 when jaw member 114 is in the clamping configuration and cutting blade is vibrating to treat tissue. In particular, tissue contacting surface 115 vibrates with cutting blade 117 as a result of the relatively resilient (or compliant) nature of layer 125 of laminate liner.

During use of one particular example of the instrument 2, tissue may be positioned between the jaw member 114 and cutting blade 117. Subsequently, trigger 7 may be depressed to activate the cutting blade 117 to treat tissue of interest.

As cutting blade 117 vibrates against tissue contacting surface 115 of jaw member 214 and treats tissue, the relatively flexible (or compliant) nature of layer 125 of laminate liner allows tissue contacting surface 115 to also vibrate, which reduces shear stresses against tissue contacting surface 115 caused by the vibrations of cutting blade 117.

The unique configuration of jaw member 114 including laminate liner 119 thereon allows the cutting blade 117 to treat tissue for prolonged periods of time without the likelihood of the tissue contacting surface 115 wearing or breaking down. As a result thereof, the operative life of the jaw member 115 (and/or cutting blade 117) is increased when compared to jaw members (and/or cutting blades) associated with conventional ultrasonic instruments.

With reference to Fig. 4, a jaw member 214 according to an alternate example is illustrated. Jaw member 214 is configured for use with instrument 2 and configured to function substantially similar to that of jaw members 14, 114. In view thereof, only those features unique to jaw member 214 are described herein.

One or more synchronizing devices 219 is operably coupled to jaw housing 213 and tissue contacting surface 215 and is configured to facilitate moving tissue contacting surface 215 in concert with cutting blade 217 when cutting blade 217 is treating tissue. For example, in particular embodiments one or more hydraulic mechanisms, spring mechanisms and/or pneumatic mechanisms (or combination thereof) may be used as synchronizing devices 219 that operable coupled to jaw housing 213 and are configured to support tissue contacting surface 215 thereon such that tissue contacting surface 215 is free-floating.

During use of one particular example of the instrument 2, tissue may be positioned between the jaw member 214 and cutting blade 217. Subsequently, trigger 7 may be depressed to activate the cutting blade 217 to treat tissue of interest. As cutting blade 217 vibrates against tissue contacting surface 215 of jaw member 214 and treats tissue, the free-floating nature of tissue contacting surface 215 allows tissue contacting surface 215 to also vibrate in general synchronization with cutting blade 217, which reduces shear stresses against tissue contacting surface 215 caused by the vibrations of cutting blade 217.

The unique configuration of jaw member 214 including a free-floating tissue contacting surface 215 allows the cutting blade 217 to treat tissue for prolonged periods of time without the likelihood of the tissue contacting surface 215 wearing or breaking down. As a result thereof, the operative life of the jaw member 214 (and/or cutting blade 217) is increased when compared to jaw members (and/or cutting blades) associated with conventional ultrasonic instruments.

In the instance where hydraulic and pneumatic mechanisms are utilized as the synchronizing device to provide a free-floating tissue contacting surface 215, one or more fluid sources and fluid lines (not explicitly shown) may be provided with instrument 2 and configured to provided one more suitable fluids, e.g., liquid, gas, etc. to the hydraulic and pneumatic mechanisms. In the instance where a spring mechanism is utilized as the synchronizing device to provide a free-floating tissue contacting surface 215, one or more springs, e.g., torsional springs, coil springs, compression springs, may be coupled between jaw housing 213 and tissue contacting surface 215.

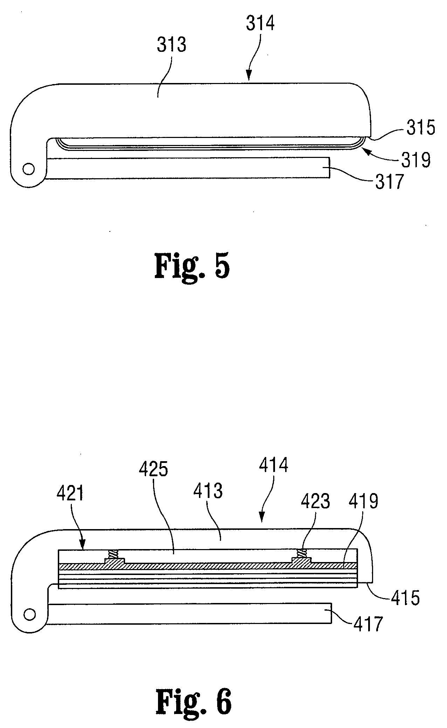

With reference to Fig. 5, a jaw member 314 according to an embodiment is illustrated. Jaw member 314 is configured for use with instrument 2 and configured substantially similar to that of jaw members 14, 114. In view thereof, only those features unique to jaw member 314 are described herein.

Unlike jaw members 14, 114 that include a single laminate liner 19, 119, respectively, jaw member 314 includes a plurality of laminate liners 319. Each laminate liner 319 may be formed similar to the aforementioned laminate liners 19, 119. Unlike laminate liners 19, 119 which are fixedly coupled to respective jaw housings 13, 113, each laminate liner 319 is selectively removable from tissue contacting surface 315. Accordingly, when one of the laminate liners 319 is worn or damaged as a result of prolonged use of cutting blade 317, a user may remove the worn or damaged laminate liner 319 from the tissue contacting surface 315. Each of the laminate liners 319 may be coupled to tissue contacting surface 315 (or each other) by one or more suitable coupling methods, e.g., adhesives, brazing, soldering, welding, and so forth.

In one particular embodiment (Fig. 6), a laminate liner cartridge 421 may be operably coupled to jaw member 414 and configured to house a plurality of laminate liners 419 therein. In this embodiment, one or more springs 423 may be operably coupled to a laminate liner plate 425 provided in laminate cartridge 421 and configured to bias the plurality of laminate liners 419 outwardly from cartridge 421 to facilitate dispensing each laminate liner 419 from cartridge 421. Laminate liners 419 are selectively removable from cartridge 421 and may be coupled to cartridge 421 (or each other) by one or more suitable coupling methods, e.g., adhesives, brazing, soldering, welding, and so forth. As can be appreciated, cartridge 421 facilitates maintaining the laminate liners 419 in a relatively fixed orientation during operation of cutting blade 417. In addition, spring(s) 423 may provide a free-floating tissue contacting surface 415.

As with laminate liner 19, as cutting blades 317, 417 vibrate against tissue contacting surfaces 315, 415 of jaw members 314, 414 and treats tissue, the relatively flexible (or compliant) nature of the laminate liners 319, 419 reduce shear stresses against tissue contacting surfaces 315, 415 caused by the vibrations of cutting blades 317, 417. Further, the aforementioned advantages of instrument 2 utilizing laminate liner 19 are equally obtainable with laminate liners 319, 419.

It is contemplated that second jaw member 27 may be used with any of the aforementioned examples or interchangeable with different configurations of jaw members 14, 114, 214, 314, 414.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

## Claims

1. An ultrasonic surgical instrument (2), comprising:
a housing (6) having an elongated shaft (4) extending therefrom, the shaft defining a longitudinal axis therethrough and having a jaw member disposed at a distal end thereof, the jaw member movable between an open configuration and a clamping configuration, the jaw member including a tissue contacting surface (315, 415) having a laminate liner (319, 419) disposed thereon, and
a cutting blade (317, 417) extending from a distal end of the shaft and operably coupled to the housing and adjacent the jaw member to treat tissue **characterized in that** there are a plurality of, laminate liners stacked upon one another and selectively removable from one another;

2. The ultrasonic surgical instrument according to claim 1, wherein the plurality of laminate liners includes at least one substrate (21) and at least one low durometer, low friction material.

3. The ultrasonic surgical instrument according to claim 2, wherein the at least one substrate is coated with the at least one low durometer, low friction material.

4. The ultrasonic surgical instrument according to claim 2 or 3, wherein the at least one substrate is formed from a material selected from the group consisting of metal and ceramic.

5. The ultrasonic surgical instrument according to claim 1, wherein the plurality of laminate liners includes at least one low durometer, low friction material.

6. The ultrasonic surgical instrument according to claim 2, 3, 4 or 5, wherein the at least one low durometer, low friction material is selected from the group consisting of polytetrafluoroethylene and silicone.

7. The ultrasonic surgical instrument according to any preceding claim, wherein the plurality of laminate liners is configured to facilitate moving the tissue contacting surface of the jaw member in concert with the cutting blade when the cutting blade is treating tissue.

8. The ultrasonic surgical instrument according to any preceding claim, wherein at least one resilient member (423) is operably coupled to the jaw member and configured to bias the plurality of laminate liners in a generally outwardly configuration to facilitate removing each laminate liner of the plurality of liners.

9. The ultrasonic surgical instrument according to claim 8, wherein the at least one resilient member includes a spring.

## Patentansprüche

1. Chirurgisches Ultraschallinstrument (2), umfassend:
ein Gehäuse (6), das einen länglichen Schaft (4) aufweist, der sich davon erstreckt, wobei der Schaft eine Längsachse dort hindurch definiert und ein Klemmbackenelement aufweist, das an einem distalen Ende davon angeordnet ist, wobei das Klemmbackenelement zwischen einer offenen Konfiguration und einer Klemmkonfiguration bewegbar ist, wobei das Klemmbackenelement eine Gewebekontaktfläche (315, 415) mit einer darauf angeordneten Laminat-Decklage (319, 419) aufweist; und
eine Schneidklinge (317, 417), die sich von einem distalen Ende des Schaftes erstreckt und betriebsfähig mit dem Gehäuse und benachbart zu dem Klemmbackenelement gekoppelt ist, um Gewebe zu behandeln, **dadurch gekennzeichnet, dass** eine Mehrzahl von Laminat-Decklagen aufeinander gestapelt und selektiv voneinander entfernbar sind.

2. Chirurgisches Ultraschallinstrument gemäß Anspruch 1, wobei die Mehrzahl der Laminat-Decklagen mindestens ein Substrat (21) und mindestens ein reibungsarmes Material mit geringer Härte umfassen.

3. Chirurgisches Ultraschallinstrument gemäß Anspruch 2, wobei das mindestens eine Substrat mit dem mindestens einen reibungsarmen Material mit geringer Härte beschichtet ist.

4. Chirurgisches Ultraschallinstrument gemäß Anspruch 2 oder 3, wobei das mindestens eine Substrat aus einem Material gebildet ist, das ausgewählt ist aus der Gruppe, bestehend aus Metall und Keramik.

5. Chirurgisches Ultraschallinstrument gemäß Anspruch 1, wobei die Mehrzahl der Laminat-Decklagen mindestens ein reibungsarmes Material mit geringer Härte umfasst.

6. Chirurgisches Ultraschallinstrument gemäß Anspruch 2, 3, 4 oder 5, wobei das mindestens eine reibungsarme Material mit geringer Härte ausgewählt ist aus der Gruppe, bestehend aus Polytetrafluorethylen und Silikon.

7. Chirurgisches Ultraschallinstrument gemäß einem der vorhergehenden Ansprüche, wobei die Mehrzahl der Laminat-Decklagen konfiguriert ist, das Bewegen der Gewebekontaktfläche des Klemmbackenelements in Übereinstimmung mit der Schneidklinge zu erleichtern, wenn die Schneidklinge das Gewebe bearbeitet.

8. Chirurgisches Ultraschallinstrument gemäß einem der vorhergehenden Ansprüche, wobei mindestens ein elastisches Element (423) betriebsfähig mit dem Klemmbackenelement gekoppelt und zum Vorspannen von der Mehrzahl der Laminat-Decklagen in einer im Allgemeinen nach außen gerichteten Konfiguration konfiguriert ist, um das Entfernen jeder Laminat-Decklage von der Mehrzahl der Decklagen zu erleichtern.

9. Chirurgisches Ultraschallinstrument gemäß Anspruch 8, wobei das mindestens eine elastische Element eine Feder umfasst.

## Revendications

1. Instrument chirurgical ultrasonore (2), comprenant :
un boîtier (6) ayant un arbre allongé (4) s'étendant de celui-ci, l'arbre définissant un axe longitudinal qui le traverse et ayant un élément de mors à son extrémité distale, l'élément de mors étant mobile entre une configuration ouverte et un configuration de serrage, l'élément de mors comprenant une surface (315, 415) venant en contact avec le tissu et ayant un revêtement stratifié (319, 419) qui est disposé sur celle-ci et
une lame de coupe (317, 417) s'étendant d'une extrémité distale de l'arbre et couplée en service au boîtier et adjacente à l'élément de mors pour traiter le tissu, **caractérisé en ce qu'**il y a une pluralité de revêtements stratifiés qui sont empilés l'une sur l'autre et peuvent être séparés sélectivement l'un de l'autre.

2. Instrument chirurgical ultrasonore selon la revendication 1, dans lequel la pluralité de revêtements stratifiés comprennent au moins un substrat (21) et au moins un matériau de faible frottement et de faible dureté.

3. Instrument chirurgical ultrasonore selon la revendication 2, dans lequel le au moins un substrat est revêtu du au moins un matériau de faible frottement et de faible dureté.

4. Instrument chirurgical ultrasonore selon la revendication 2 ou 3, dans lequel le au moins un substrat est formé d'un matériau choisi dans le groupe constitué des métaux et des céramiques.

5. Instrument chirurgical ultrasonore selon la revendication 1, dans lequel la pluralité de revêtements stratifiés comprennent au moins un matériau de faible frottement et de faible dureté.

6. Instrument chirurgical ultrasonore selon la revendication 2, 3, 4 ou 5, dans lequel le au moins un matériau de faible frottement et de faible dureté est choisi dans le groupe constitué du polytétrafluoroéthylène et d'une silicone.

7. Instrument chirurgical ultrasonore selon l'une quelconque des revendications précédentes, dans lequel la pluralité de revêtements stratifiés sont configurés pour faciliter le déplacement de la surface de l'élément de mors venant en contact avec le tissu conjointement avec la lame de coupe lorsque la lame de coupe traite le tissu.

8. Instrument chirurgical ultrasonore selon l'une quelconque des revendications précédentes, dans lequel au moins un élément élastique (423) est couplé en service à l'élément de mors et est configuré pour presser la pluralité de revêtements stratifiés en configuration généralement extérieure pour faciliter le retrait de chaque revêtement stratifié de la pluralité de revêtements.

9. Instrument chirurgical ultrasonore selon la revendication 8, dans lequel le au moins un élément élastique comprend un ressort.
